# EUROPEAN PATENT SPECIFICATION

(11) **EP 0 523 330 B1**
(45) Date of publication and mention of the grant of the patent: **20.03.1996**
(21) Application number: 92107278.1
(22) Date of filing: 29.04.1992
(51) Int. Cl.: A61K 9/00

(54) **Implant compositions containing a biologically active protein, peptide or polypeptide**
Implantationzusammensetzungen, die biologisch aktive Proteine, Peptide oder Polypeptide enthalten
Compositions d'implantation contenant des protéines, peptides ou polypeptides biologiquement actifs

(30) Priority: 24.06.1991 US 719898
(43) Date of publication of application: 20.01.1993
(73) Proprietor: AMERICAN CYANAMID COMPANY, Wayne, NJ 07470-8426 (US)
(72) Inventor: Steber, William David, Ledgewood, New Jersey 07852 (US); Cady, Susan Mancini, Yardley, Pennsylvania 19067 (US); Johnson, David Farley, P.O.Box 60, Stamford, Conn.06904-0060 (US); Rice, Theresa, Newton, Pennsylvania 18940 (US)
(74) Representative: Wächtershäuser, Günter, Prof. Dr.

(56) References cited:
- EP-A- 0 246 540
- EP-A- 0 403 032
- WO-A-92/02211

## Description

The need for and the difficulties encountered in the development of methods and compositions which continuously release pharmaceutical preparations in a uniform manner over extended periods of time are well known.

The present invention relates to an implantble composition for the parenteral administration of an essentially uniform and continuous amount of a biologically active protein, peptide or polypeptide over an extended period of time which comprises a compacted, indented and partially coated composition containing from one to three layers each of which contains on a weight basis from about 20% to 80% of a biologically active protein, peptide or polypeptide, about 10% to 75% of a fat or wax or mixture thereof, 0% to about 20% of a buffer or salt or mixture thereof and 0% to about 25% of a sugar.

Surprisingly, it has been found that increased blood levels of biologically active proteins, peptides and polypeptides may be obtained and maintained for extended periods of time by implanting animals with the compacted, indented and partially coated composition of the present invention.

The implantable composition of the present invention is preferably a compacted, indented and partially coated composition containing from one to three layers each of which contains on a weight basis from about 20% to 80% of a biologically active protein, peptide or polypeptide, about 10% to 75% of a fat or wax or mixture thereof, about 1% to 20% of a buffer or salt or mixture thereof and about 1% to 25% of a sugar.

A more preferred implantable composition of this invention is a compacted, indented and partially coated composition containing from one to three layers each of which contains on a weight basis from about 35% to 70% of a biologically active protein, peptide or polypeptide, about 15% to 50% of a fat or wax or mixture thereof, about 1% to 10% of a buffer or salt or mixture thereof and about 5% to 15% of a sugar.

Biologically active proteins, peptides and polypeptides suitable for administration in the composition of the present invention include somatotropins, somatomedins, growth factor, and other biologically active fragments and derivatives thereof. Preferred proteins include porcine, ovine, equine, bovine, avian and human somatotropins; and is meant to encompass those which are of natural, synthetic, recombinant or biosynthetic origin. More preferred proteins are somatotropins with alterations in the α-helix 3 region, α-helix 2 region, combinations thereof and in combination with other mutations with E34 rpST, I122L + E34 rpST and A6TS11R + E34 rpST being most preferred.

Waxes and fats which are suitable for use in the composition of the present invention in general have melting points higher than 40°C. The wax of the invention may be defined as a low-melting organic mixture or compound of high molecular weight, solid at room temperature and generally similar in composition to fats and oils except that it contains no glycerides. Some are hydrocarbons; others are esters of fatty acids and alcohols. These compounds include saturated or unsaturated long chain C₁₀-C₂₄ fatty acids, alcohols, esters, salts, ethers or mixtures thereof. They are classed among the lipids. Waxes are thermoplastic, but since they are not high polymers, they are not considered in the family of plastics. Common properties are water repellency; smooth texture; nontoxicity; freedom from objectionable odor and color. They are combustible and have good dielectric properties. They are soluble in most organic solvents and are insoluble in water.

The major types are as follows:
I. Natural
   1. Animal (beeswax, lanolin, shellac wax, Chinese insect wax)
   2. Vegetable (carnauba, candelilla, bayberry, sugarcane)
   3. Mineral
      (a) Fossil or earth waxes (ozocerite, ceresin, montan)
      (b) Petroleum waxes (paraffin, microcyrystalline slack or scale wax)
II. Synthetic
   1. Ethylenic polymers and polyol ether-esters ("Carbowax")
   2. Chlorinated naphthalenes ("Halowax")
   3. Hydrocarbon type via Fischer-Tropsch synthesis

The fat of the present invention may be defined as a glyceryl ester of higher fatty acids such as myristic, stearic and palmitic. Such esters and their mixtures are solids at room temperatures and exhibit crystalline structure. Lard and tallow are examples. There is no chemical difference between a fat and an oil, the only distinction being that fats are solid at room temperature and oils are liquid. The term "fat" usually refers to triglycerides specifically, whereas "lipid" is all-inclusive.

The fat is preferably composed of mono-, di- or triglyceryl esters of long chain C₁₀-C₂₄ fatty acids. The mono-, di-, or triglycerides are composed predominantly of myristates, stearates, palmitates, laurates, linoleates, linolenates, oleates, and residues or mixtures thereof, having melting points greater than 50°C, being most preferred. Glyceryl trimyristate is a most preferred fat.

Sugars suitable for use in the composition of the present invention include mono-, di- or trisaccharides such as glucose, mannose, sorbitol, mannitol, lactose, sucrose, maltose, cellobiose and raffinose. Preferred sugars are non-reducing mono-, di- or trisaccharides with sucrose, raffinose, sorbitol and mannitol being most preferred.

Buffers are added to the implant compositions of the present invention to adjust the pH of the composition to a value of from about 6.0 to 8.5 in order to effect the solubility of the somatotropin and consequently the release of the somatotropin from the implant composition. Buffers suitable for use in the compositions of the invention include sodium and potassium phosphates, borates, carbonates, glycinates and the like or mixtures thereof with a mixture of monobasic sodium phosphate and dibasic sodium phosphate being preferred to adjust the pH of the compositions to a preferred value of from about 6.5 to 8.0.

Salts suitable for use in the composition of this invention include salts such as sodium chloride or potassium chloride.

Additives such as stabilizers, preservatives, surfactants or mixtures thereof may be included in the compositions of the invention. Preferred stabilizers include dehydroacetic acid, salicylanilide, sorbic acid, boric acid, benzoic acid, and salts thereof; hydroxypropyl cellulose, hydroxypropyl methylcellulose, sodium nitrite and sodium nitrate. The amounts of said additives suitable for use in the invention range from about 0.1% to 20% on a weight basis.

Surprisingly, it has been found that increased blood levels of somatotropins may be obtained and maintained for extended periods of time by implanting animals with the composition of the invention. Elevated blood levels of the biologically active proteins, peptides and polypeptides are generally observed and associated with beneficial and/or therapeutic effects. The effects include weight gain, increased growth rate, improved feed efficiency, decreased body fat, improved lean meat to fat ratio, improved muscle size and increased milk production in lactating animals. Maintaining the elevated blood levels is an indication of the slow release of the active ingredient. Properties such as increased growth rate, improved feed efficiency, increased lean meat and increased milk production are generally observed when elevated blood levels of the active ingredient is maintained. The invention includes the use of the compositions herein to increase growth rate, improve feed efficiency, increase lean meat in animals, improve milk production and increase and maintain levels of somatotropins in the blood stream of animals.

Implantable composition of the present invention useful for the administration of a biologically active protein, peptide or polypeptide may be prepared by incorporating the active ingredient, buffer or salt or mixture thereof and sugar with a molten fat, wax or mixture thereof to obtain a coarse powder. Compacted and indented compositions are then prepared with a tablet press set up with conventional implant sizes such as 3,97 mm, 3,18 mm (5/32, 1/8 inches) using a special top punch. The top punch has a tapered projection on the center line of the punch which will form a conical indentation in the composition when compressed. One to three layers of the coarse powder is then placed into the die and compressed with the special top punch to form compacted and indented compositions. In a preferred embodiment of the invention the amount of biologically active protein, peptide or polypeptide present in each layer increases away from the indentation. The compacted and indented compositions are then coated with one or two layers of a semipermeable material to form the implantable compositions of the invention. The indentation remains essentially uncoated and becomes a passageway for the active ingredient to exit the composition of the invention over an extended period of time.

Semipermeable materials suitable for coating the compressed and indented compositions of the present invention include semipermeable polymers such as methacrylate ester copolymers, ethyl cellulose polymers and the like. Additives such as plasticizers and fillers may be added to the semipermeable polymers in amounts of from 1% to 20% on a weight basis with triethyl citrate and talc being a preferred plasticizer and filler respectively. The thickness of each coating surrounding the compacted and indented compositions is from about 0,0127 mm (0.5 mils) to 0,635 mm (25 mils).

In order to facilitate a further understanding of the invention, the following examples are presented to illustrate more specific details thereof. The invention is not to be limited thereby except as defined in the claims.

### EXAMPLE 1

### Preparation of implant compositions for the parenteral administration of somatotropins

1. Preparation of somatotropin, sugar, buffer and additives in a size range suitable for incorporation in the fat, wax or mixture thereof by spray drying nay be accomplished by dissolving the somatotropin and sugar in water and then adding the desired buffer solution such as a 1:2 mixture of monobasic and dibasic sodium phosphate. Additives such as hydroxypropyl cellulose may be added and allowed to dissolve. The solution is then spray-dried in a Buchi mini spray drier, model #190.
2. Preparation of granular powder. A homogeneous mixture of the spray dried powder in the molten fat, wax or mixture thereof is prepared and the resulting mixture is cooled to give a powder. The powder is tableted using a Stokes model #512 tablet press set up with 1.59 cm (5/8 inch) punches and dies. These tablets are milled using a benchtop Glen Mills MicrohammerMill to form a coarse granular powder.
3. Preparation of compacted and indented compositions. Layered, compacted and indented compositions are made with a Stokes model #521 single tablet press set up with a 3,97 mm (5/32 inch) die and special top punch. The top punch has a 3mm tapered projection on the center line of the punch. The base of the projection is about 1mm. To make layered, compacted and indented compositions, the inner end granular powder is placed into the die first and lightly tamped, then the indent end granular powder is placed into the die. The press is operated by hand, so that each implant is made one at a time. To make uniform implants, a 3,18 mm (1/8 inch) die and special top punch is used. The top punch has a 3 mm tapered projection on the center line of the punch and the base of the projection is about 1 mm. The desired amount of granular powder is placed into the die and the uniform, compacted and indented compositions are prepared by operating the press by hand.
4. Preparation of partially coated implant compositions. The compacted and indented compositions are coated with one or two layers of a semipermeable polymeric material using a MINI HI-COATER® (trademark of Vector Laboratories). The surface within the indentation remains essentially uncoated and becomes a passageway for the active ingredient to exit the composition of the invention over an extended period of time.

Utilizing the above procedure with the materials listed in Table I below yields the implant compositions listed in Table II below.

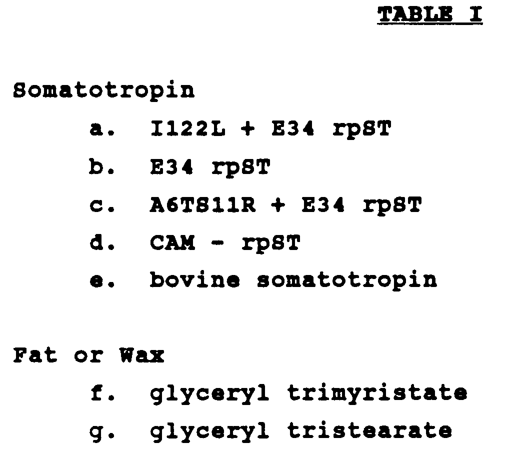

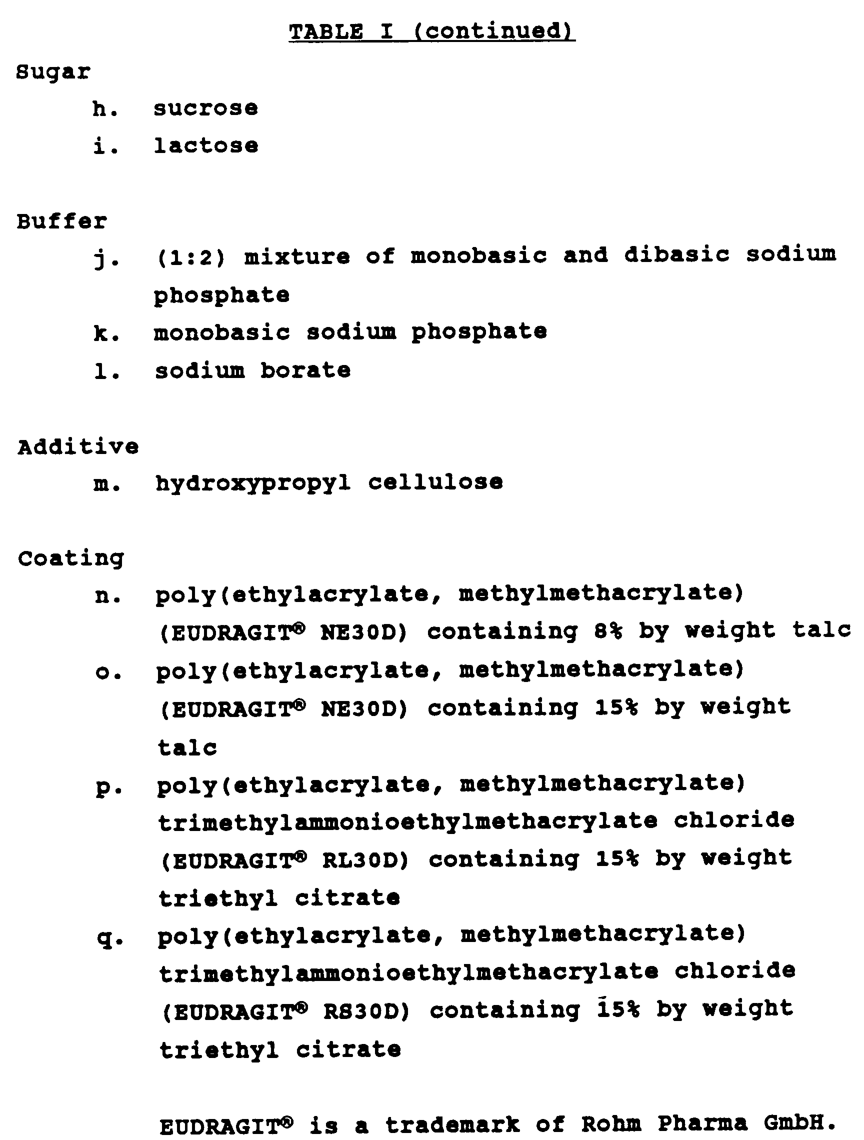

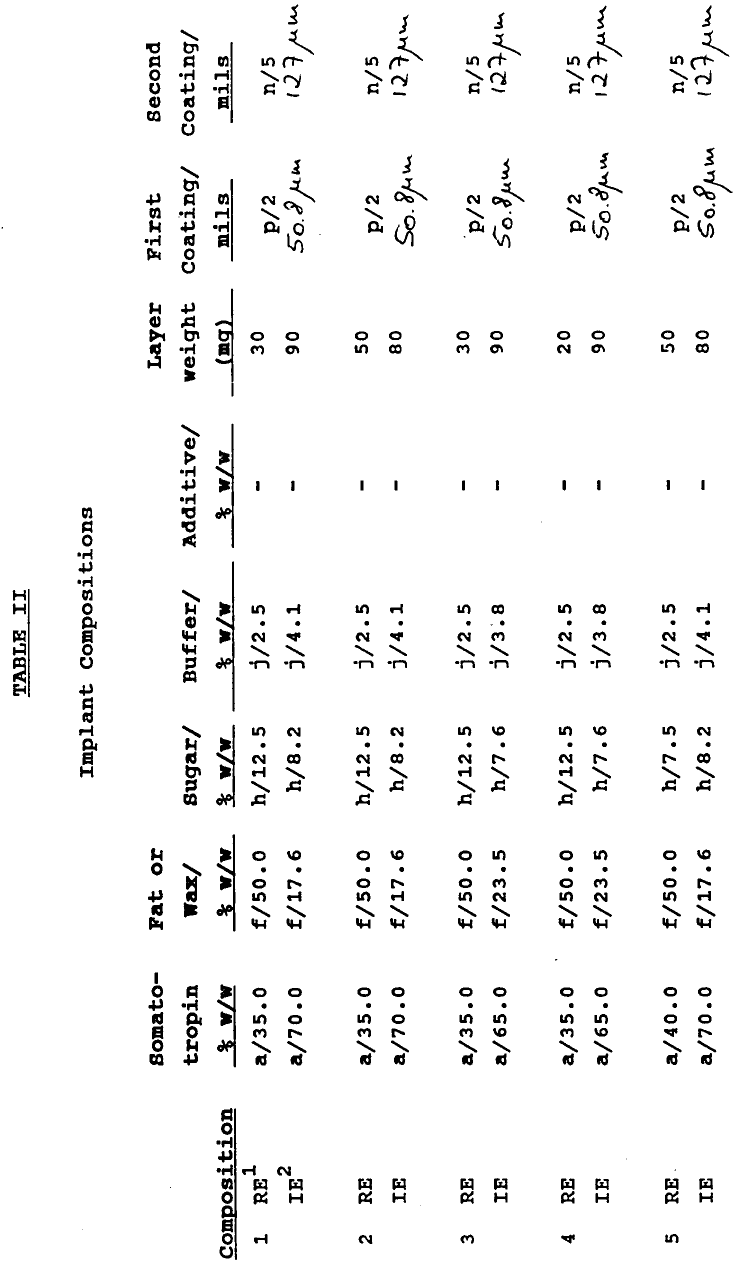

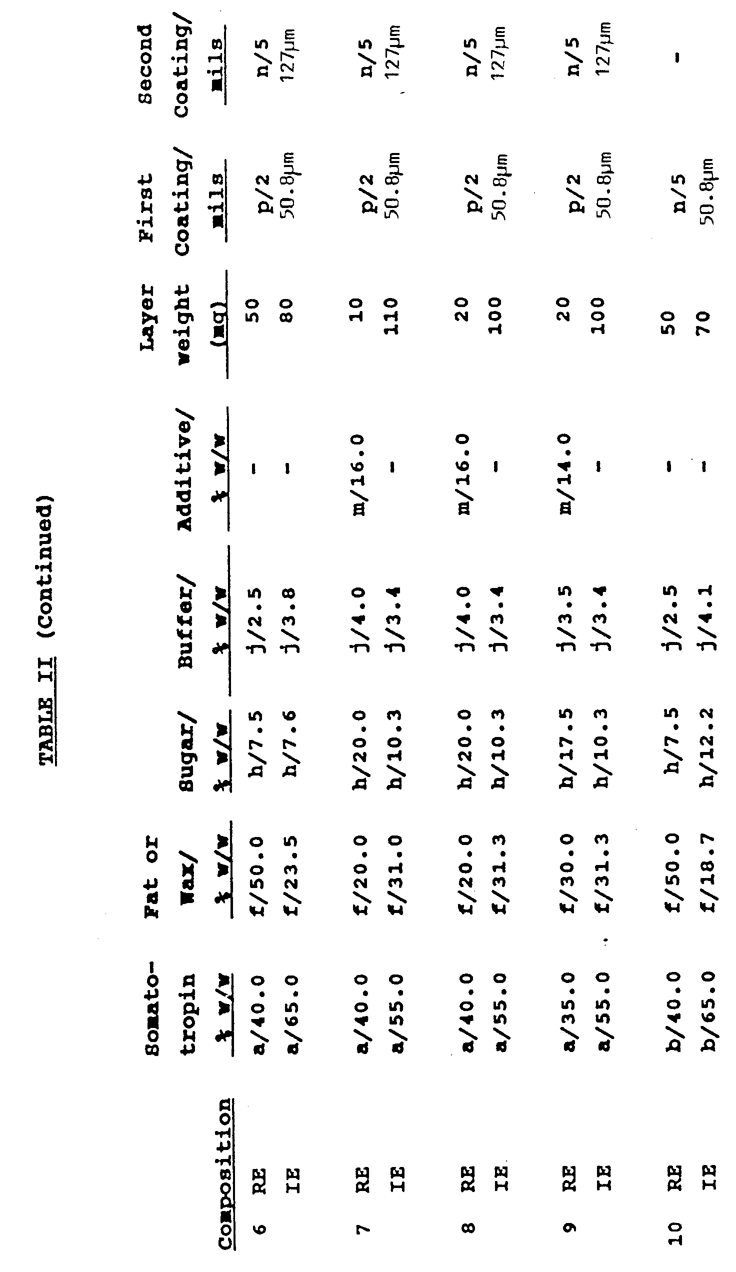

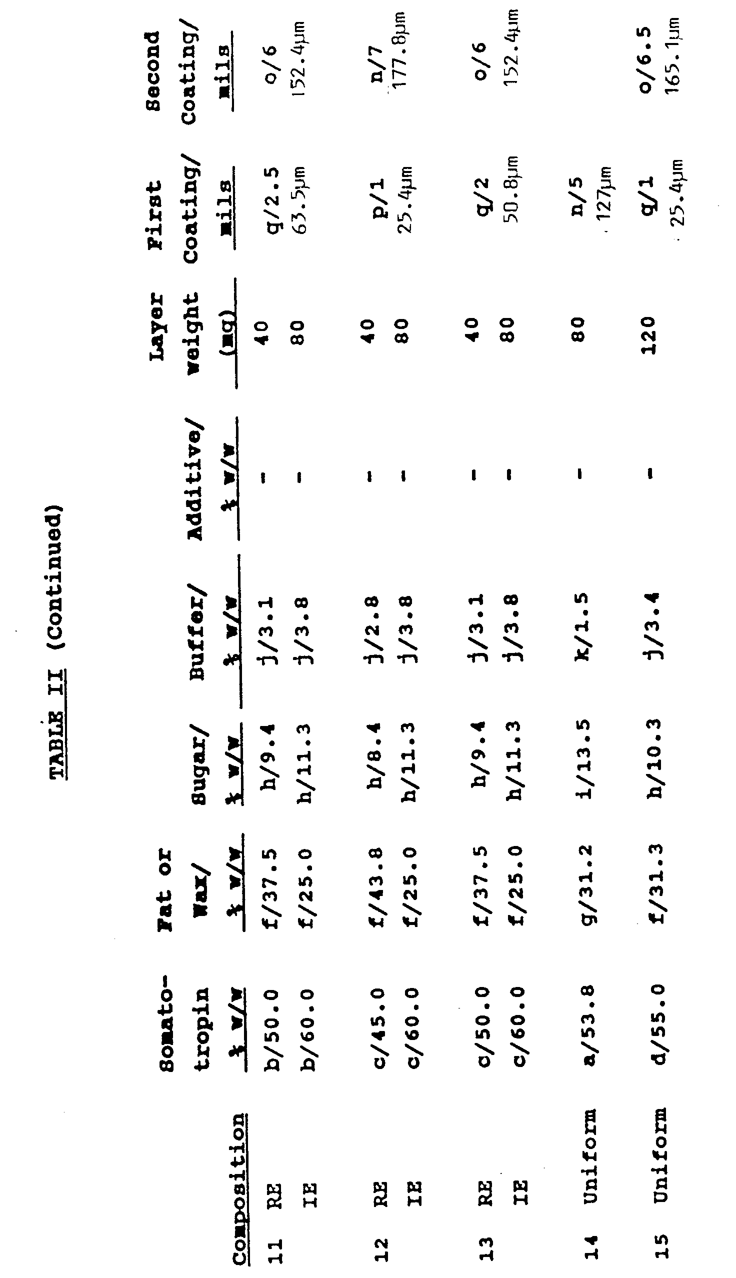

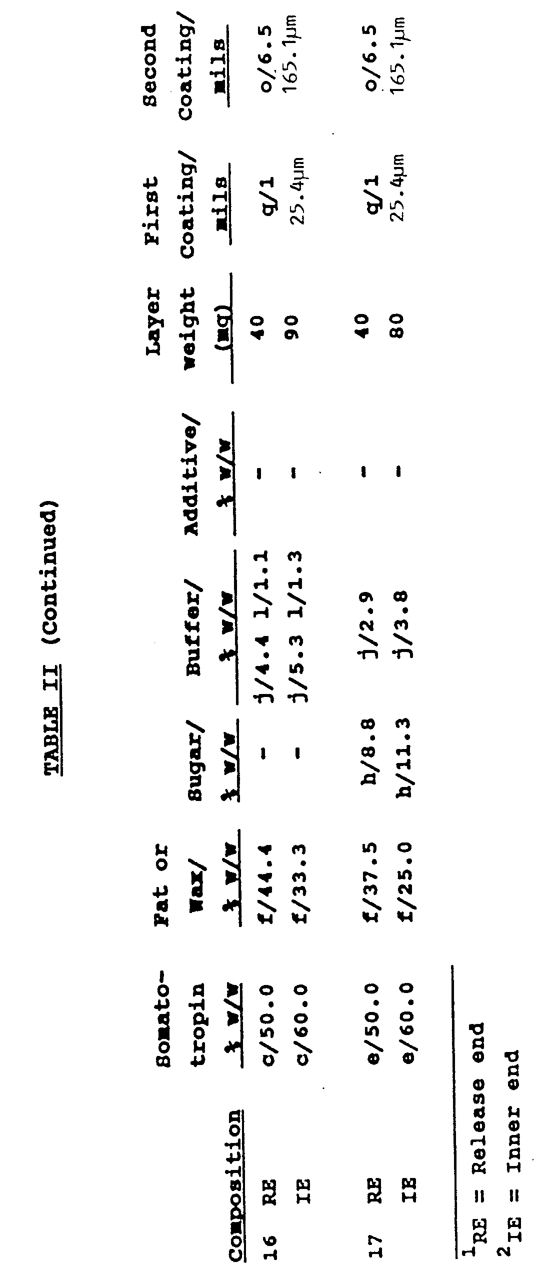

### EXAMPLE 2

### Sustained release of compositions of the invention in pigs

Pigs are divided into groups of four animals. Throughout the test, all pigs are fed the same ration containing 20% crude protein. The pigs are not treated for three days and daily porcine somatotropin blood levels are obtained for each group of animals. Then two implants, listed in Table II, are implanted in the ear of each pig. Somatotropin levels in the blood of the animals is determined by standard RIA techniques daily. The results of this experiment, summarized in Table III below, demonstrate the effectiveness of the compositions of the invention for increasing and maintaining elevated somatotropin levels in the blood for extended periods of time.

**TABLE III**

| **Average Plasma rpST Concentration (ng/mL by Radioimmunoassay) for Pig Experiments** | | | | | | |
|---|---|---|---|---|---|---|
| **Time (Days)** | **Composition from Table II** | | | | | |
| | **2** | **7** | **10** | **11** | **12** | **13** |
| -3 | 1.9 | 1.4 | 1.5 | 2.1 | 3.8 | 2.5 |
| -2 | 0.9 | 2.2 | 2.6 | 4.9 | 2.4 | 3.8 |
| -1 | 1.2 | 2.3 | 1.7 | 2.4 | 3.3 | 4.2 |
| 1 | 6.6 | 5.5 | 4.9 | 1.7 | 3.0 | 1.4 |
| 2 | 8.2 | 3.5 | 35.2 | 1.5 | 3.5 | 1.6 |
| 3 | 9.8 | 13.9 | 97.1 | 2.6 | 3.8 | 3.2 |
| 4 | 6.3 | 11.3 | 60.9 | 1.9 | 3.2 | 2.4 |
| 5 | 3.4 | 17.6 | 56.4 | 1.5 | 18.9 | 8.6 |
| 6 | 20.1 | 16.2 | 474.8 | 11.2 | 61.2 | 17.6 |
| 7 | 26.4 | 20.6 | 242.6 | 3.3 | 40.3 | 22.9 |
| 8 | 26.1 | 20.0 | 110.6 | 118.1 | 35.4 | 41.3 |
| 9 | 50.0 | 19.4 | 70.7 | 87.0 | 19.7 | 43.0 |
| 10 | 42.9 | 25.9 | 45.4 | 56.6 | 30.0 | 55.0 |
| 11 | 26.1 | 35.7 | 82.4 | 44.3 | 15.7 | 32.5 |
| 12 | 16.0 | 34.2 | 47.1 | 34.8 | 22.7 | 35.3 |
| 13 | 26.2 | 29.8 | 52.1 | 40.1 | 6.7 | 25.0 |
| 14 | 19.9 | 31.4 | 28.8 | 54.4 | 8.0 | 39.8 |
| 15 | 10.2 | 25.3 | 21.3 | 25.9 | 4.8 | 20.0 |
| 16 | 8.8 | 20.7 | 19.7 | 31.3 | 4.7 | 12.5 |
| 17 | 13.4 | 10.5 | 6.8 | 29.2 | 4.9 | 10.2 |
| 18 | 8.0 | 15.4 | 7.8 | 18.5 | 4.0 | 10.0 |
| 19 | 32.1 | 34.4 | 10.0 | 16.1 | 3.8 | 5.3 |
| 20 | 5.8 | 9.4 | 8.2 | 14.9 | 2.8 | 6.8 |
| 21 | 9.6 | 13.6 | 9.9 | 8.7 | 2.7 | 3.1 |
| 22 | 15.1 | 11.2 | 5.7 | 7.4 | 35.6 | 7.1 |
| 23 | 60.8 | 7.1 | 6.0 | 15.9 | 12.8 | 4.3 |
| 24 | 4.9 | 7.6 | 12.6 | 11.2 | 4.0 | 4.4 |
| 25 | 8.4 | 7.0 | 5.4 | 5.4 | 2.3 | 3.4 |
| 26 | 8.9 | 6.6 | 4.0 | 5.3 | 2.7 | 6.8 |
| 27 | 6.7 | 7.3 | 4.8 | 8.6 | 5.0 | 6.5 |

### EXAMPLE 3

### In Vitro dissolution evaluation of implants

Two implants are placed in a plastic tube containing 10 mL of a phosphate buffer solution (pH 7.4, 100 mM NaCl, 50mM Na₂HPO₄/NaH₂PO₄, 0.2% Na azide) and the tube is placed in a water bath where the temperature of the water in the unit is maintained at 39°C. The tube is kept in the water bath for two days, then the solution is removed from the tube and analyzed for the appropriate somatotropin by HPLC and the solution is discarded. New phosphate buffer solution is added to the tube and the tube is placed into the water bath for three additional days and analyzed as described above. This procedure is repeated several times at various time intervals until the experiment is terminated. Table IV below summarizes the release rates of the appropriate somatotropin for several compositions listed in Table II.

**TABLE IV**

| **Release Rate (mg/day)** | | | |
|---|---|---|---|
| **Days** | **Composition** | | |
| | **2** | **7** | **10** |
| 0 - 2 | 0.6 | 0.6 | 1.0 |
| 2 - 5 | 3.8 | 7.6 | 11.4 |
| 5 - 9 | 7.2 | 7.0 | 6.9 |
| 9 - 12 | 5.3 | 3.4 | 4.2 |
| 12 - 16 | 2.7 | 1.7 | 2.1 |
| 16 - 19 | 1.6 | 1.3 | 1.8 |
| 19 - 23 | 1.3 | 0.9 | 1.4 |
| 23 - 28 | 0.9 | 0.6 | 1.0 |

Following the above procedure but analyzing the solutions for the appropriate somatotropin at different time intervals than described above gives the release rates summarized below in Tables V, VI and VII.

**TABLE V**

| **Release Rate (mg/day)** | | |
|---|---|---|
| **Days** | **Composition** | |
| | **11** | **13** |
| 0 - 1 | 0.1 | 0.0 |
| 1 - 2 | 2.5 | 1.4 |
| 2 - 5 | 7.0 | 3.9 |
| 5 - 9 | 5.9 | 6.4 |
| 9 - 12 | 3.9 | 4.3 |
| 12 - 16 | 2.5 | 2.5 |
| 16 - 19 | 1.7 | 1.3 |
| 19 - 22 | 1.4 | 1.1 |
| 22 - 26 | 1.0 | 1.1 |
| 26 - 28 | 0.8 | 0.6 |

**TABLE VI**

| **Release Rate (mg/day)** | |
|---|---|
| **Days** | **Composition** |
| | **12** |
| 0 - 1 | 0.5 |
| 1 - 3 | 2.0 |
| 3 - 7 | 5.1 |
| 7 - 10 | 7.1 |
| 10 - 14 | 3.9 |
| 14 - 17 | 1.9 |
| 17 - 21 | 1.3 |
| 21 - 24 | 0.9 |
| 24 - 28 | 0.7 |

**TABLE VII**

| **Release Rate (mg/day)** | | | |
|---|---|---|---|
| **Days** | **Composition** | | |
| | **15** | **16** | **17** |
| 0 - 1 | 0.0 | 0.0 | 0.0 |
| 1 - 2 | 2.2 | 0.7 | 0.7 |
| 2 - 5 | 1.5 | 3.0 | 3.3 |
| 5 - 9 | 3.9 | 4.4 | 4.5 |
| 9 - 12 | 4.6 | 4.1 | 2.6 |
| 12 - 16 | 2.4 | 2.3 | 1.1 |
| 16 - 20 | 1.5 | 1.6 | 0.7 |
| 20 - 23 | 0.9 | 1.2 | 0.4 |
| 23 - 26 | 0.9 | 1.1 | 0.3 |

Additionally, following the above procedure but using three implants gives the release rates summarized below in Table VIII.

**TABLE VIII**

| **Release Rate (mg/day)** | |
|---|---|
| **Days** | **Composition** |
| | **14** |
| 0 - 1 | 0.0 |
| 1 - 4 | 1.4 |
| 4 - 7 | 3.9 |
| 7 - 11 | 4.7 |
| 11 - 14 | 3.3 |
| 14 - 18 | 1.9 |
| 18 - 21 | 1.5 |
| 21 - 34 | 0.8 |

## Claims (Claims for the following Contracting State(s): AT, BE, CH, LI, DE, DK, FR, GB, IT, LU, NL, PT, SE)

1. An implantable composition for the parenteral administration of an essentially uniform and continuous amount of a biologically active protein, peptide or polypeptide over an extended period of time characterized by a compacted, indented and partially coated composition containing from one to three layers each of which contains on a weight basis from 20% to 80% of a biologically active protein, peptide or polypeptide, 10% to 75% of a fat or wax or mixture thereof, 0% to 20% of a buffer or salt or mixture thereof and 0% to 25% of a sugar, and wherein the identation remains essentially uncoated and acts as a passageway for the biologically active protein, peptide or polypeptide.

2. The composition according to claim 1 wherein the biologically active protein, peptide or polypeptide is selected from the group consisting of somatotropins, somatomedins, and growth factors, including porcine, ovine, equine, bovine, avian and human somatotropins; the fat is selected from the group consisting of glyceryl trisyristate, glyceryl tripalmitate and glyceryl tristearate, the buffer is selected from the group consisting of sodium borate, sodium carbonate, monobasic sodium phosphate, dibasic sodium phosphate and mixtures thereof, the sugar is selected from the group consisting of glucose, mannose, sucrose, raffinose, sorbitol, mannitol and lactose; the coating comprises one or two layers of a semipermeable material and wherein said composition optionally is characterized by a stabilizer, a surfactant or mixtures thereof.

3. The composition according to claim 2 wherein the porcine somatotropin is selected from the group consisting of E34 rpST, I122L + E34 rpST and A6TS11R + E34 rpST.

4. The composition according to claim 2 wherein the buffer is a mixture of monobasic sodium phosphate and dibasic sodium phosphate.

5. The composition according to claim 2 wherein the semipermeable material is a methacrylate ester copolymer containing on a weight basis from 1% to 20% ethyl citrate or talc and the thickness of each coating is from 0.0127 mm (0.5 mils) to 0.635 mm (25 mils).

6. The composition according to claim 1 wherein the amount of biologically active protein, peptide or polypeptide present in each layer increases away from the indentation.

7. A process for the preparation of an implantable composition according to one of claims 1 to 6, characterized by
(a) mixing the biologically active protein, peptide or polypeptide with a molten fat, wax or mixture thereof and optionally with a buffer, salt or sugar to form a coarse powder;
(b) compacting one to three layers of the coarse powder to form a compacted mass;
(c) punching the compacted mass with a tapered projection to form a tapered indentation in the compacted mass; and
(d) coating the indented compacted mass with a semipermeable material such that a passageway is formed by the indentation from the compacted mass through the coating.

8. The process according to claim 7 wherein step (b) and step (c) occur simultaneously.

9. The process according to claim 7 wherein the compacted mass is characterized by the composition of claim 1.

10. The process according to claim 7 wherein step (a) is further characterized by
(a) dissolving the biologically active protein, peptide or polypeptide, and buffer or salt or mixture thereof, in water to form a solution;
(b) spray drying the solution to form a spray dried powder;
(c) dissolving the spray dried powder in the molten fat, wax or mixture thereof to form a homogeneous mixture;
(d) cooling the homogeneous mixture to form a powder;
(e) compressing the powder to form a compressed mass; and
(f) milling the compressed mass to form a coarse granular powder.

## Claims (Claims for the following Contracting State(s): ES, GR)

1. A process for the preparation of an implantable composition for the parenteral administration of an essentially uniform an continuous amount of a biologically active protein, peptide or polypeptide over an extended period of time having a compacted, indented and partially coated composition containing from one to three layers each of which contains on a weight basis from 20% to 80% of a biologically active protein, peptide of polypeptide, 10% to 75% of a fat or wax or mixture thereof, 0% to 20% of a buffer or salt or mixture thereof and 0% to 25% of a sugar, and wherein the indentation remains essentially uncoated and acts as a passageway for the biologically active protein, peptide or polypeptide, characterized by
(a) mixing the biologically active protein, peptide or polypeptide with a molten fat, wax or mixture thereof and optionally with a buffer, salt or sugar to form a coarse powder;
(b) compacting one to three layers of the coarse powder to form a compacted mass;
(c) punching the compacted mass with a tapered projection to form a tapered indentation in the compacted mass; and
(d) coating the indented compacted mass with a semipermeable material such that a passageway is formed by the indentation from the compacted mass through the coating.

2. The process according to Claim 1, wherein step (b) and step (c) occur simultaneously.

3. The process according to Claim 1, wherein step (a) is further characterized by
(a) dissolving the biologically active protein, peptide or polypeptide and buffer or salt or mixture thereof, in water to form a solution;
(b) spray drying the solution to form a spray dried powder;
(c) dissolving the spray dried powder in the molten fat, wax or mixture thereof to form a homogeneous mixture;
(d) cooling the homogeneous mixture to form a powder;
(e) compressing the powder to form a compressed mass; and
(f) milling the compressed mass to form a coarse granular powder.

4. The process according to Claim 1, wherein the biologically active protein,
peptide or polypeptide is selected from the group consisting of somatotropins, somatomedins, and growth factors, including porcine, ovine, equine, bovine, avian and human somatotropins; the fat is selected from the group consisting of glyceryl trisyristate, glyceryl tripalmitate and glyceryl tristearate, the buffer is selected from the group consisting of sodium borate, sodium carbonate, monobasic sodium phosphate, dibasic sodium phosphate and mixtures thereof, the sugar is selected from the group consisting of glucose, mannose, sucrose, raffinose, sorbitol, mannitol and lactose; the coating comprises one or two layers of a semipermeable material and wherein said composition optionally comprises a stabilizer, a surfactant or mixtures thereof.

5. The process according to Claim 4, wherein the porcine somatotropin is selected from the group consisting of E34 rpST, I122L + E34 rpST and A6TS11R + E34 rpST.

6. The process according to Claim 4, wherein the buffer is a mixture of monobasic sodium phosphate and dibasic sodium phosphate.

7. The process according to Claim 4, wherein the semipermeable material is a methacrylate ester copolymer containing on a weight basis from 1% to 20% ethyl citrate or talc and the thickness of each coating is from 0.0127 mm (0.5 mils) to 0.635 mm (25 mils).

8. The process according to Claim 1, wherein the amount of biologically active protein, peptide or polypeptide present in each layer increases away from the indentation.

## Patentansprüche (Patentansprüche für folgende(n) Vertragsstaat(en): AT, BE, CH, LI, DE, DK, FR, GB, IT, LU, NL, PT, SE)

1. Implantierbare Zusammensetzung für die parenterale Verabreichung einer im Wesentlichen gleichförmigen und kontinuierlichen Menge eines biologisch aktiven Proteins, Peptids oder Polypeptids über einen ausgedehnten Zeitraum, gekennzeichnet durch eine kompaktierte, eingekerbte und teilweise beschichtete Zusammensetzung, die 1 bis 3 Schichten enthält, von denen jede 20 Gew.-% bis 80 Gew.-% eines biologisch aktiven Proteins, Peptids oder Polypeptids, 10 Gew.-% bis 75 Gew.-% eines Fettes oder Wachses oder einer Mischung derselben, 0 Gew.-% bis 20 Gew.-% eines Puffers oder eines Salzes oder einer Mischung derselben und 0 Gew.-% bis 25 Gew.-% eines Zuckers enthält, und worin die Einkerbung im wesentlichen unbeschichtet bleibt und als Durchtrittsweg für das biologisch aktive Protein, Peptid oder Polypeptid wirkt.

2. Zusammensetzung nach Anspruch 1, in der das biologisch aktive Protein, Peptid oder Polypeptid aus der Gruppe ausgewählt ist, die aus Somatotropinen, Somatomedinen und Wachstumsfaktoren, einschließlich Schweine-, Schafs-, Pferde-, Rinder-, Vogel- und Human-Somatotropinen besteht; das Fett aus der Gruppe ausgewählt ist, die aus Glyceryltrisyristat, Glyceryltripalmitat und Glyceryltristearat besteht, der Puffer aus der Gruppe ausgewählt ist, die aus Natriumborat, Natriumcarbonat, einbasigem Natriumphosphat, zweibasigem Natriumphosphat und Mischungen derselben besteht, der Zucker aus der Gruppe ausgewählt ist, die aus Glukose, Mannose, Saccharose, Raffinose, Sorbit, Mannit und Laktose besteht; die Beschichtung ein oder zwei Schichten aus einem semipermeablen Material umfaßt und worin die Zusammensetzung gegebenenfalls durch einen Stabilisator, ein Tensid oder Mischungen derselben gekennzeichnet ist.

3. Zusammensetzung nach Anspruch 2, in der das Schweine-Somatotropin aus der Gruppe ausgewählt ist, die aus E34 rpST, I122L + E34 rpST und A6TS11R + E34 rpST besteht.

4. Zusammensetzung nach Anspruch 2, in der der Puffer eine Mischung von einbasigem Natriumphosphat und zweibasigem Natriumphosphat ist.

5. Zusammensetzung nach Anspruch 2, in der das semipermeable Material ein Methacrylatester-Copolymer ist, das 1 Gew.-% bis 20 Gew.-% Ethylcitrat oder Talkum enthält, und die Dicke jeder Beschichtung 0,0127 mm (0,5 Mil) bis 0,635 mm (25 Mil) beträgt.

6. Zusammensetzung nach Anspruch 1, in der die Menge an biologisch aktivem Protein, Peptid oder Polypeptid, die in jeder Schicht vorhanden ist, mit der Entfernung von der Einkerbung zunimmt.

7. Verfahren zur Herstellung einer implantierbaren Zusammensetzung nach einem der Ansprüche 1 bis 6, dadurch gekennzeichnet, daß man
a) das biologisch aktive Protein, Peptid oder Polypeptid mit einem geschmolzenen Fett, Wachs oder einer Mischung derselben und gegebenenfalls mit einem Puffer, Salz oder Zucker mischt, um ein grobes Pulver zu bilden;
b) 1 bis 3 Schichten des groben Pulvers kompaktiert, um eine kompaktierte Masse zu bilden;
c) die kompaktierte Masse mit einem sich verjüngenden Vorsprung prägt, um in der kompaktierten Masse eine sich verjüngende Einkerbung zu bilden; und
d) die eingekerbte kompaktierte Masse mit einem semipermeablen Material derart beschichtet, daß durch die Einkerbung ein Durchtrittsweg aus der kompaktierten Masse durch die Beschichtung gebildet wird.

8. Verfahren nach Anspruch 7, in dem Schritt b) und Schritt c) gleichzeitig stattfinden.

9. Verfahren nach Anspruch 7, in dem die kompaktierte Masse durch die Zusammensetzung nach Anspruch 1 gekennzeichnet ist.

10. Verfahren nach Anspruch 7, in dem der Schritt a) weiter dadurch gekennzeichnet ist, daß man
a) das biologisch aktive Protein, Peptid oder Polypeptid und Puffer oder Salz oder die Mischung derselben in Wasser löst, um eine Lösung zu bilden;
b) die Lösung sprühtrocknet, um ein sprühgetrocknetes Pulver zu bilden;
c) das sprühgetrocknete Pulver in dem geschmolzenen Fett, Wachs oder der Mischung derselben löst, um eine homogene Mischung zu bilden;
d) die homogene Mischung abkühlt, um ein Pulver zu bilden;
e) das Pulver komprimiert, um eine komprimierte Masse zu bilden; und
f) die komprimierte Masse mahlt, um ein grobes granuläres Pulver zu bilden.

## Patentansprüche (Patentansprüche für folgende(n) Vertragsstaat(en): ES, GR)

1. Verfahren zur Herstellung einer implantierbaren Zusammensetzung für die parenterale Verabreichung einer im wesentlichen gleichförmigen und kontinuierlichen Menge eines biologisch aktiven Proteins, Peptids oder Polypeptids über einen ausgedehnten Zeitraum, die eine kompaktierte, eingekerbte und teilweise beschichtete Zusammensetzung aufweist, welche 1 bis 3 Schichten enthält, von denen jede 20 Gew.-% bis 80 Gew.-% eines biologisch aktiven Proteins, Peptids oder Polypeptids, 10 Gew.-% bis 75 Gew.-% eines Fettes oder Wachses oder einer Mischung derselben, 0 Gew.-% bis 20 Gew.-% eines Puffers oder eines Salzes oder einer Mischung derselben und 0 Gew.-% bis 25 Gew.-% eines Zuckers enthält, und worin die Einkerbung im wesentlichen unbeschichtet bleibt und als Durchtrittsweg für das biologisch aktive Protein, Peptid oder Polypeptid wirkt, dadurch gekennzeichnet, daß man
a) das biologisch aktive Protein, Peptid oder Polypeptid mit einem geschmolzenen Fett, Wachs oder einer Mischung derselben und gegebenenfalls mit einem Puffer, Salz oder Zucker mischt, um ein grobes Pulver zu bilden;
b) 1 bis 3 Schichten des groben Pulvers kompaktiert, um eine kompaktierte Masse zu bilden;
c) die kompaktierte Masse mit einem sich verjüngenden Vorsprung prägt, um in der kompaktierten Masse eine sich verjüngende Einkerbung zu bilden; und
d) die eingekerbte kompaktierte Masse mit einem semipermeablen Material derart beschichtet, daß durch die Einkerbung ein Durchtrittsweg aus der komprimierten Masse durch die Beschichtung gebildet wird.

2. Verfahren nach Anspruch 1, in dem Schritt b) und Schritt c) gleichzeitig stattfinden.

3. Verfahren nach Anspruch 1, in dem der Schritt a) weiter dadurch gekennzeichnet ist, daß man
a) das biologisch aktive Protein, Peptid oder Polypeptid und Puffer oder Salz oder die Mischung derselben in Wasser löst, um eine Lösung zu bilden;
b) die Lösung sprühtrocknet, um ein sprühgetrocknetes Pulver zu bilden;
c) das sprühgetrocknete Pulver in dem geschmolzenen Fett, Wachs oder der Mischung derselben löst, um eine homogene Mischung zu bilden;
d) die homogene Mischung abkühlt, um ein Pulver zu bilden;
e) das Pulver komprimiert, um eine komprimierte Masse zu bilden; und
f) die komprimierte Masse mahlt, um ein grobes granuläres Pulver zu bilden.

4. Verfahren nach Anspruch 1, in dem das biologisch aktive Protein, Peptid oder Polypeptid aus der Gruppe ausgewählt ist, die aus Somatotropinen, Somatomedinen und Wachstumsfaktoren, einschließlich Schweine-, Schafs-, Pferde-, Rinder-, Vogel- und Human-Somatotropinen besteht; das Fett aus der Gruppe ausgewählt ist, die aus Glyceryltrisyristat, Glyceryltripalmitat und Glyceryltristearat besteht, der Puffer aus der Gruppe ausgewählt ist, die aus Natriumborat, Natriumcarbonat, einbasigem Natriumphosphat, zweibasigem Natriumphosphat und Mischungen derselben besteht, der Zucker aus der Gruppe ausgewählt ist, die aus Glukose, Mannose, Saccharose, Raffinose, Sorbit, Mannit und Laktose besteht; die Beschichtung ein oder zwei Schichten aus einem semipermeablen Material umfaßt und worin die Zusammensetzung gegebenenfalls durch einen Stabilisator, ein Tensid oder Mischungen derselben gekennzeichnet ist.

5. Verfahren nach Anspruch 4, in dem das Schweine-Somatotropin aus der Gruppe ausgewählt ist, die aus E34 rpST, I122L + E34 rpST und A6TS11R + E34 rpST besteht.

6. Verfahren nach Anspruch 4, in dem der Puffer eine Mischung von einbasigem Natriumphosphat und zweibasigem Natriumphosphat ist.

7. Verfahren nach Anspruch 4, in dem das semipermeable Material ein Methacrylatester-Copolymer ist, das 1 Gew.-% bis 20 Gew.-% Ethylcitrat oder Talkum enthält, und die Dicke jeder Beschichtung 0,0127 mm (0,5 Mil) bis 0,635 mm (25 Mil) beträgt.

8. Verfahren nach Anspruch 1, in dem die Menge an biologisch aktivem Protein, Peptid oder Polypeptid, die in jeder Schicht vorhanden ist, mit der Entfernung von der Einkerbung zunimmt.

## Revendications (Revendications pour l'(les) Etat(s) contractant(s) suivant(s): AT, BE, CH, LI, DE, DK, FR, GB, IT, LU, NL, PT, SE)

1. Composition implantable pour l'administration parentérale, pendant une durée prolongée, d'une quantité essentiellement uniforme et continue d'une protéine, d'un peptide ou d'un polypeptide biologiquement actif, caractérisée par une composition compactée, comportant une encoche et un revêtement partiel, contenant de une à trois couches, dont chacune contient, rapporté en poids, de 20% à 80% d'une protéine, d'un peptide ou d'un polypeptide biologiquement actif, de 10% à 75% d'une graisse ou cire, ou d'un mélange de celles-ci, de 0% à 20% d'un tampon ou d'un sel ou d'un mélange de ceux-ci et de 0% à 25% d'un sucre, et dans laquelle l'encoche demeure pratiquement sans revêtement et joue le rôle de voie de passage pour la protéine, le peptide ou le polypeptide biologiquement actif.

2. Composition selon la revendication 1, dans laquelle la protéine, le peptide ou le polypeptide biologiquement actif est choisi parmi des somatotropines, somatomédines, et facteurs de croissance, comprenant des somatotropines porcines, ovines, équines, bovines, aviaires et humaines; la graisse est choisie parmi le trimyristate de glycéryle, le tripalmitate de glycéryle et le tristéarate de glycéryle, le tampon est choisi parmi le borate de sodium, le carbonate de sodium, le phosphate de sodium monobasique, le phosphate de sodium dibasique et des mélanges de ceux-ci, le sucre est choisi parmi le glucose, le mannose, le saccharose, le raffinose, le sorbitol, le mannitol et le lactose; le revêtement comprend une ou deux couches d'un matériau semi-perméable, et dans laquelle ladite composition est éventuellement caractérisée par un stabilisant, un tensioactif ou des mélanges de ceux-ci.

3. Composition selon la revendication 2, dans laquelle la somatotropine porcine est choisie parmi E34 rpST, I122L + E34 rpST et A6TS11R + E34 rpST.

4. Composition selon la revendication 2, dans laquelle le tampon est un mélange de phosphate de sodium monobasique et de phosphate de sodium dibasique.

5. Composition selon la revendication 2, dans laquelle le matériau semi-perméable est un copolymère d'ester de méthacrylate contenant, rapporté en poids, de 1% à 20% de citrate d'éthyle ou de talc, et l'épaisseur de chaque couche de revêtement est comprise entre 0,0127 mm (0,5 millième de pouce) et 0,635 mm (25 millièmes de pouce).

6. Composition selon la revendication 1, dans laquelle la quantité de protéine, peptide ou polypeptide biologiquement actif présent dans chaque couche augmente lorsque l'on s'éloigne de l'encoche.

7. Procédé de préparation d'une composition implantable selon l'une des revendications 1 à 6, caractérisé par les étapes consistant à
(a) mélanger la protéine, le peptide ou le polypeptide biologiquement actif avec de la graisse ou cire fondue ou un mélange de celles-ci et éventuellement avec un tampon, un sel ou un sucre pour constituer une poudre grossière;
(b) compacter une à trois couches de cette poudre grossière afin de constituer une masse compactée;
(c) poinçonner cette masse compactée avec un élément en saillie conique afin de constituer une encoche conique dans la masse compactée; et
(d) revêtir la masse compactée présentant une encoche avec un matériau semi-perméable de telle façon qu'une voie de passage soit formée par l'encoche à partir de la masse compactée à travers le revêtement.

8. Procédé selon la revendication 7, dans lequel l'étape (b) et l'étape (c) se produisent simultanément.

9. Procédé selon la revendication 7, dans lequel la masse compactée est caractérisée par la composition de la revendication 1.

10. Procédé selon la revendication 7, dans lequel l'étape (a) est en outre caractérisée par le fait de
(a) dissoudre la protéine, le peptide ou le polypeptide biologiquement actif, et le tampon ou le sel ou un mélange de ceux-ci, dans de l'eau pour constituer une solution;
(b) sécher la solution par pulvérisation pour former une poudre séchée par pulvérisation;
(c) dissoudre la poudre séchée par pulvérisation dans la graisse ou cire fondue ou un mélange de celles-ci afin de constituer un mélange homogène;
(d) refroidir le mélange homogène pour former une poudre;
(e) comprimer la poudre pour constituer une masse comprimée; et
(f) broyer la masse comprimée pour former une poudre granulaire grossière.

## Revendications (Revendications pour l'(les) Etat(s) contractant(s) suivant(s): ES, GR)

1. Procédé de préparation d'une composition implantable pour l'administration parentérale, pendant une durée prolongée, d'une quantité essentiellement uniforme et continue d'une protéine, d'un peptide ou d'un polypeptide biologiquement actif, comprenant une composition compactée, comportant une encoche et un revêtement partiel, contenant de une à trois couches, dont chacune contient, rapporté en poids, de 20 % à 80 % d'une protéine, d'un peptide ou d'un polypeptide biologiquement actif, de 10 % à 75 % d'une graisse ou cire ou d'un mélange de celles-ci, de 0 % à 20 % d'un tampon ou d'un sel ou d'un mélange de ceux-ci, et de 0 % à 25 % d'un sucre, et dans laquelle l'encoche demeure pratiquement sans revêtement et joue le rôle de voie de passage pour la protéine, le peptide ou le polypeptide biologiquement actif, caractérisé par les étapes consistant à
(a) mélanger la protéine, le peptide ou le polypeptide biologiquement actif avec de la graisse ou de la cire fondue ou un mélange de celles-ci et éventuellement avec un tampon, un sel ou un sucre pour constituer une poudre grossière ;
(b) compacter une à trois couches de cette poudre grossière afin de constituer une masse compactée ;
(c) poinçonner cette masse compactée avec un élément en saillie conique afin de constituer une encoche conique dans la masse compactée ; et
(d) revêtir la masse compactée présentant une encoche avec un matériau semi-perméable de telle façon qu'une voie de passage soit formée par l'encoche à partir de la masse compactée à travers le revêtement.

2. Procédé selon la revendication 1, dans lequel l'étape (b) et l'étape (c) se produisent simultanément.

3. Procédé selon la revendication 1, dans lequel l'étape (a) est en outre caractérisée par le fait de
(a) dissoudre la protéine, le peptide ou le polypeptide biologiquement actif, et le tampon ou le sel ou un mélange de ceux-ci, dans de l'eau pour constituer une solution ;
(b) sécher la solution par pulvérisation pour former une poudre séchée par pulvérisation ;
(c) dissoudre la poudre séchée par pulvérisation dans la graisse ou cire fondue ou un mélange de celles-ci afin de constituer un mélange homogène ;
(d) refroidir le mélange homogène pour former une poudre ;
(e) comprimer la poudre pour constituer une masse comprimée ; et
(f) broyer la masse comprimée pour former une poudre granulaire grossière.

4. Procédé selon la revendication 1, dans lequel la protéine, le peptide ou le polypeptide biologiquement actif est choisi parmi des somatotropines, somatomédines, et facteurs de croissance, comprenant des somatotropines porcine, ovine, équine, bovine, aviaire et humaine ; la graisse est choisie parmi le trimyristate de glycéryle, le tripalmitate de glycéryle et le tristéarate de glycéryle, le tampon est choisi parmi le borate de sodium, le carbonate de sodium, le phosphate de sodium monobasique, le phosphate de sodium dibasique et les mélanges de ceux-ci, le sucre est choisi parmi le glucose, le mannose, le saccharose, le raffinose, le sorbitol, le mannitol et le lactose ; le revêtement comprend une ou deux couches d'un matériau semi-perméable, et dans lequel ladite composition comprend éventuellement un stabilisant, un tensioactif ou des mélanges de ceux-ci.

5. Procédé selon la revendication 4, dans lequel la somatotropine porcine est choisie parmi E34 rpST, I122L + E34 rpST et A6TS11R + E34 rpST.

6. Procédé selon la revendication 4, dans lequel le tampon est un mélange de phosphate de sodium monobasique et de phosphate de sodium dibasique.

7. Procédé selon la revendication 4, dans lequel le matériau semi-perméable est un copolymère d'ester de méthacrylate contenant, rapporté en poids, de 1 % à 20 % de citrate d'éthyle ou de talc, et l'épaisseur de chaque couche de revêtement est comprise entre 0,0127 mm (0,5 millièmes de pouce) et 0,635 mm (25 millièmes de pouce).

8. Procédé selon la revendication 1, dans lequel la quantité de protéine, peptide ou polypeptide biologiquement actif présent dans chaque couche augmente lorsque l'on s'éloigne de l'encoche.
